# EUROPEAN PATENT SPECIFICATION

(11) **EP 1 221 988 B1**
(45) Date of publication and mention of the grant of the patent: **29.03.2006**
(21) Application number: 00969699.8
(22) Date of filing: 20.10.2000
(51) Int. Cl.: A61L 17/00, A61B 17/06

(54) **SUTURE MATERIAL**
MEDIZINISCHES NÄHMATERIAL
MATERIEL DE SUTURE

(30) Priority: 20.10.1999 GB 9924694
(43) Date of publication of application: 17.07.2002
(73) Proprietor: Tyco Healthcare Group LP, Mansfield, MA 02048 (US)
(72) Inventor: HEALY, David Michael, Ayr KA7 4EG (GB); GILCHRIST, Thomas, Ayr KA7 2TW (GB)
(74) Representative: Ouzman, Beverley Nicola Claire
(86) International application number: PCT/GB2000/004049
(87) International publication number: WO 2001/028601

(56) References cited:
- EP-A- 0 328 421
- EP-A- 0 633 032
- US-A- 5 413 788
- US-A- 5 534 288
- US-A- 5 744 151

## Description

The present invention relates to a suture material having antimicrobial characteristics.

Sutures are the threads or wires used to stitch two bodily surfaces together. Typically, sutures are required to close surgical incisions and to treat deep lacerations inflicted on a patient.

Suture types fall into two main categories; absorbable and non-absorbable. Additionally, the sutures can be of monofilament or multifilament structure, with the multifilament sutures being braided or twisted. A variety of sizes of sutures are available. Typical commercially available suture types are listed below:

**Non-absorbable:**

| | |
|---|---|
| Silk | twisted, braided & multifilament |
| Nylon polyamide | monofilament |
| Polypropylene | monofilament |
| Polyester | braided multifilament |
| PTFE | monofilament |
| PVDF | monofilament |
| Stainless steel | monofilament |
| Linen | multifilament |

**Absorbable:**

| | |
|---|---|
| PGA | monofilament & multifilaments |
| PLA | monofilament & multifilaments |
| Lactide/Glycolide | |
| Copolymers | monofilaments & multifilaments |
| Catgut | monofilament |
| Collagen | monofilament |

In general braided multifilaments have a smoother surface than the alternative twisted multifilaments and so remain more cohesive when stitched. Monofilaments, being formed from a single fibre, cannot unravel and thus lose cohesiveness.

To improve the lubrication along the surface of the suture and to provide friction to improve knot strength, the sutures may be coated. Conventionally however monofilament sutures are not coated. Coatings which may be applied include 100% beeswax BP, Silicone, PTFE (e.g. Teflon), PVP, polylactic acid (PLA), polyglycolactide (PLG), polycaprolactones and copolymers thereof. Often the coatings will incorporate detergents or other lubricating substances, e.g. calcium stearate.

However, sutures used for surgical wound closure are associated with increased bacterial infectivity. Sutures draw contaminants into the wound closure and provide a surface along which micro-organisms can track as a biofilm. Contamination of the wound via the suture can arise from the local environment (particularly in gut surgery), the closure area around the wound, inappropriate handling of the suture or from contaminated suture stock.

Antimicrobial compositions comprising a soluble silver compound deposited on a support material such as titania for use on or in sutures or other medical devices are known from US 5,413,788.

It is an object of the present invention to reduce the risk of infection due to suturing a wound, by providing sutures having antimicrobial characteristics.

Thus, in one aspect, the present invention provides a surgical suture material having either:
a) an external surface at least partially coated with an anti-microbial composition comprising a water-soluble metal ion-releasing glass as an anti-microbial agent; or
b) a water-soluble metal ion-releasing glass as an anti-microbial agent incorporated therein.

The surgical suture material may be formed from any suitable substance and may be absorbable or non-absorbable. Mention may be made of silk, polyester, nylon, polypropylene, polyvinylidenefluoride, linen, steel wire, catgut (beef serosa or ovine submucosa), polyglycolactide, polyamide (e.g. polyamide nylon), fibroin, polyglycolic acid and copolymers thereof. The sutures may be monofilament or may be braided or twisted multifilament yarns.

The anti-microbial composition if to be applied as a coating may be applied to the suture surface in the same way as a conventional coating. Indeed, a conventional coating material admixed with or including an anti-microbial agent is suitable for use in the present invention.

Preferably the anti-microbial agent is biodegradable over a period of time compatible with the timescale of wound healing. A slow-release of the anti-microbial active ingredient of the agent over a period of weeks or months is thus desirable.

The anti-microbial agent is a water-soluble metal ion-releasing glass, especially in particle (e.g. fine powder) form that may be simply admixed with a conventional coating and applied to the suture material. Advantageously the metal released by the glass is silver.

Thus we have found that by incorporating a comminuted anti-microbial water soluble metal ion-releasing glass either into the suture material itself or coated onto the external surface thereof, the infectivity of a wound site is reduced, whilst the handling characteristics (knotability and insertion lubricity) are maintained. Phosphorous pentoxide (P₂O₅) is preferably used as the glass former of the biodegradable glass used in the coating.

Generally the mole percentage of phosphorous pentoxide in the glass composition is less than 85%, preferably less than 60% and especially between 30-60%.

Alkali metals, alkaline earth metals and lanthanoid oxides or carbonates are preferably used as glass modifiers. Generally, the mole percentage of alkali metals, alkaline earth metals and lanthanoid oxides or carbonates is less than 60%, preferably between 40-60%.

Boron containing compounds (eg B₂O₃) are preferably used as glass additives. Generally, the mole percentage of boron containing compounds is less than 15% or less, preferably less than 5%.

Other compounds may also be added to the glass to modify its properties, for example SiO₂, Al₂O₃, SO₃, sulphate ions (SO₄²⁻), transition metal compounds (eg. first row transition metal compounds) or mixtures thereof.

Typically the soluble glasses used in this invention comprise phosphorus pentoxide (P₂O₅) as the principal glass-former, together with any one or more glass-modifying non-toxic materials such as sodium oxide (Na₂O), potassium oxide (K₂O), magnesium oxide (MgO), zinc oxide (ZnO) and calcium oxide (CaO) or mixtures thereof. The rate at which the glass dissolves in fluids is determined by the glass composition, generally by the ratio of glass-modifier to glass-former and by the relative proportions of the glass-modifiers in the glass. By suitable adjustment of the glass composition, the dissolution rates in water at 38°C ranging from substantially zero to 25mg/cm²/hour or more can be designed. However, the most desirable dissolution rate R of the glass is between 0.01 and 2.0mg/cm²/hour.

The water-soluble glass is preferably a phosphate glass, and preferably comprises a source of silver ions which may advantageously be introduced during manufacture as silver orthophosphate (Ag₃PO₄). The glass enables controlled release of silver or other metal ions, for example Zn, Cu, Mg, Ce, Mn, Bi, Se, Cs and mixtures thereof (preferably Ag, Cu, Zn and Mg and mixtures thereof) and other constituents in the glass and the content of these additives can vary in accordance with conditions of use and desired rates of release, the content of silver generally being up to 5 mole %. While we are following convention in describing the composition of the glass in terms of the mole % of oxides, of halides and of sulphate ions, this is not intended to imply that such chemical species are present in the glass nor that they are used for the batch for the preparation of the glass.

The optimum rate of release of the metal ions (eg Ag, Cu, Zn or Mg, or any of the other metal ions mentioned above) into an aqueous environment may be selected by circumstances and particularly by the specific function of the released metal ion. The invention provides a means of delivering metal ions to an aqueous medium at a rate which will maintain a concentration of metal ions in said aqueous medium of not less than 0.01 parts per million and not greater than 10 parts per million. In some cases, the required rate of release may be such that all of the metal added to the system is released in a short period of hours or days and in other applications it may be that the total metal be released slowly at a substantially uniform rate over a period extending to months or even years. In particular cases there may be additional requirements, for example it may be desirable that no residue remains after the source of the metal ions is exhausted or, in other cases, where the metal is made available it will be desirable that any materials, other than the metal itself, which are simultaneously released should be physiologically harmless. In yet other cases, it may be necessary to ensure that the pH of the resulting solution does not fall outside defined limits.

Generally, the mole percentage of these additives in the glass is less than 25%, preferably less than 10%.

In a preferred embodiment the biodegradable glass comprises 20-35 mole% Na₂O; 18-30 mole% CaO and 45-60 mole% P₂O₅.

It is a further object of the invention to provide a method of reducing the risk of infection and provide faster and more efficient healing of the wound by using the suture material of the invention to close the wound.

The present invention will now be further described by reference to the following, non-limiting, examples and to figures, in which:
- Fig. 1 :: shows the template used in the example to facilitate regular application of the suture lengths on the plates.
- Figs. 2-6 :: show digitally generated photographic images showing the results of Example 2.

### EXAMPLE 1: Suture Coating Preparation

Glasses were prepared according to Table 1.

**Table 1**

| **Annealed Solution Rate Mg.cm.**^{**-2**} **hr**^{**-1**} | **Mode µm** | **Composition** | | | | **Code** |
|---|---|---|---|---|---|---|
| | | **Na**_{**2**}**O** | **CaO** | **P**_{**2**}**O**_{**5**} | **Ag**_{**2**}**O** | |
| 0.14 | 23.71 | 22 | 26.5 | 47.0 | 4.5 | 01 |
| 1.42 | 19.44 | 33. | 16.5 | 47.0 | 3.0 | 02 |
| 0.27 | 19.96 | 27.5 | 22 | 47.0 | 3.5 | 03 |
| 1.42 | 6.50 | 33 | 16.5 | 47.0 | 3.0 | 04 |
| 16.05 | 14.02 | 30 | 10 | 47.5 | 6.5 | 05 |
| 6.02 | 12.64 | 36 | 13 | 47.5 | 3.5 | 06 |
| 3.48 | 25.44 | 34.5 | 14.5 | 47.5 | 3.5 | 07 |
| 11.28 | 12.20 | 36 | 11.5 | 47.5 | 5.0 | 08 |

These glasses were prepared as powders (mode size given in µm in Table 1 above) for incorporation into a suture coating.

### Testing

### Physical/Mechanical

It is important that addition of silver ion releasing glass into the coating does not compromise the physical or mechanical properties of the suture. The smoothness of the coating is essential in ensuring smooth insertion of the suture. The coating should not slough off on insertion and the knot properties should not be reduced. Test samples show that up to 2.5% wt/wt (final dry weight of coating) of glass powder could be added to the coating without affecting these properties and up to 5% wt/wt may be possible with some samples.

### Samples

Glass samples 01 and 04 were applied to glycolide/lactide copolymer braided multifilament sutures in a glycolide/caprolactone coating at various weights. The coat weight applied was 2% wt/wt dry weight coating onto the suture. Samples G1 to G10 contain glass 01 from 0.25-2.5% wt/wt dry weight in the coating. G11 to G20 contain glass 04 at 0.25 to 2.5% wt/wt dry weight in the coating. G21 is a nylon monofilament with 2% wt/wt coating containing 2.5% wt/wt of 04. This coating did not bond well with the suture G22 and G23 and control copolymer and control nylon sutures respectively.

### EXAMPLE 2: Anti-microbial Activity

G1 to G23 were screened against 17 test organisms.

### Suture Material

G1 to G20-Violet Polysorb size 0 sutures
G21-Dacron suture size 2/0
G22-Violet Polysorb control
G23-Dacron control

### Test Organisms

A panel of "wild-type" clinical isolates was used except for organism 5, *Staph epidermidis* NCTC 11047. This organism is a reference organism noted to be sensitive to test sutures utilised in a previous experiment.

### Gram-positive Isolates

1. *Enterococcus faecalis*
2. *Staphylococcus aureus*
3. *Enterococcus faecalis -* vancomycin resistant (VRE - VanA genotype)
4. Methicillin-resistant *Staphylococcus aureus* (MRSA - epidemic type 15)
5. *Staphylococcus epidermidis* NCTC 11047.
6. *Streptococcus agalactiae* (Group B streptococcus)

### Gram-negative Isolates

7. *Stenotrophomonas maltophilia* (formerly Xanthomonas maltophilia)
8. *Pseudomonas aeruginosa -* strain 1
9. *Pseudomonas aeruginosa -* strain 2
10. *Serratia marcescens*
11. *Enterobacter cloacae*
12. *Morganella morganii*
13. *Escherichia coli*
14. *Klebsiella pneumoniae*
15. *Acinetobacter sp.*

### Yeasts

16*. Candida albicans*
17. *Candida glabrata*

### Method

*Media -* 9 cm plates of Oxoid Iso-sensitest agar were used for all organisms except the *candida* isolates which were plated on Yeast Morphology Agar.

*Inoculum -* Overnight plate cultures of the test organisms were emulsified in physiological saline to achieve a semi-confluent growth on the agar plates.

*Inoculum procedure -* The plates were pre-dried at 37°C for 2 hours. The inoculum was applied using a sterile swab using a cross-streaking technique.

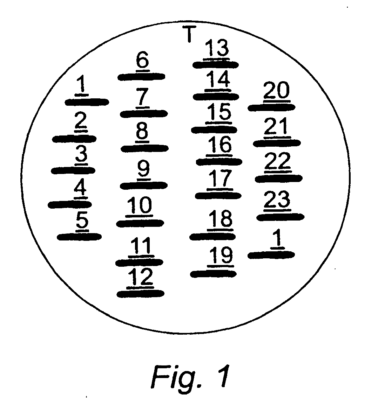

*Suture application -* The suture was cut into approximate 1 cm lengths using sterile instruments. Where possible, straight sections of suture were used. A template was constructed to facilitate regular application of the suture lengths. Each plate of test organism had the series of 21 test and 2 control sutures applied, with a replicate of suture G1 as an internal control on the far side of the plate (see Figure 1 for template). Each suture was pressed down with sterile forceps to optimise contact with the agar surface.

*Incubation* - 37°C for 18 hours. The plates were reassessed after a further 24 hours.

*Recording of results* - The maximum width of the zone of inhibition at right angles to the suture length was recorded to nearest 0.5 mm (the maximum width was recorded to avoid skewing of results due to incomplete contact of parts of the suture with the agar surface, resulting in irregular zones - see photographic results).

### Results

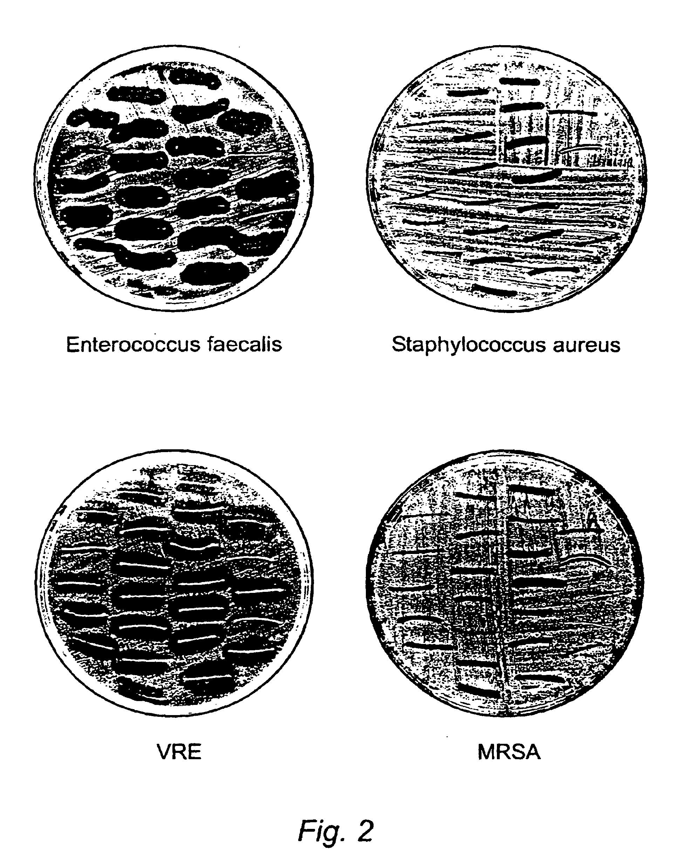

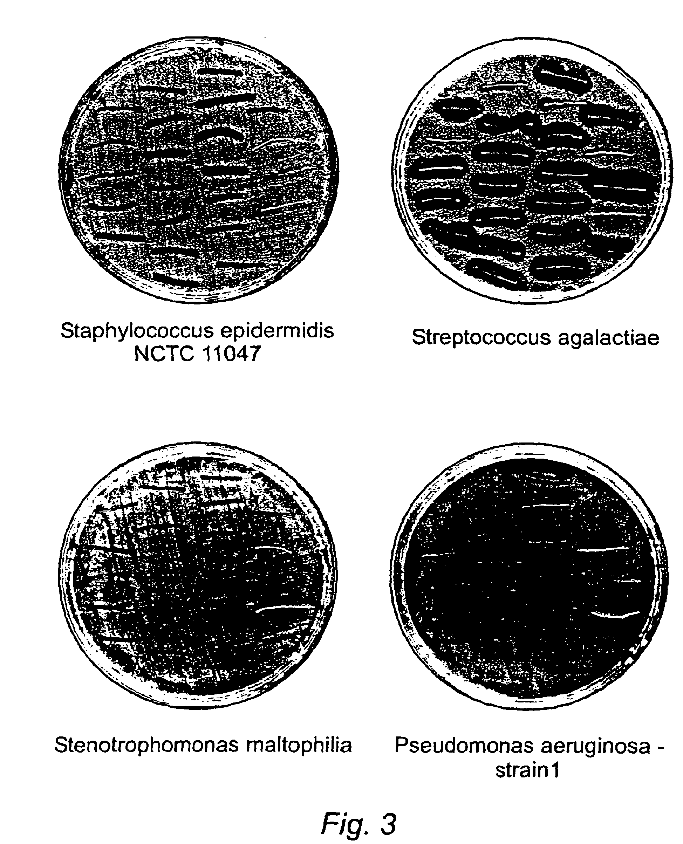

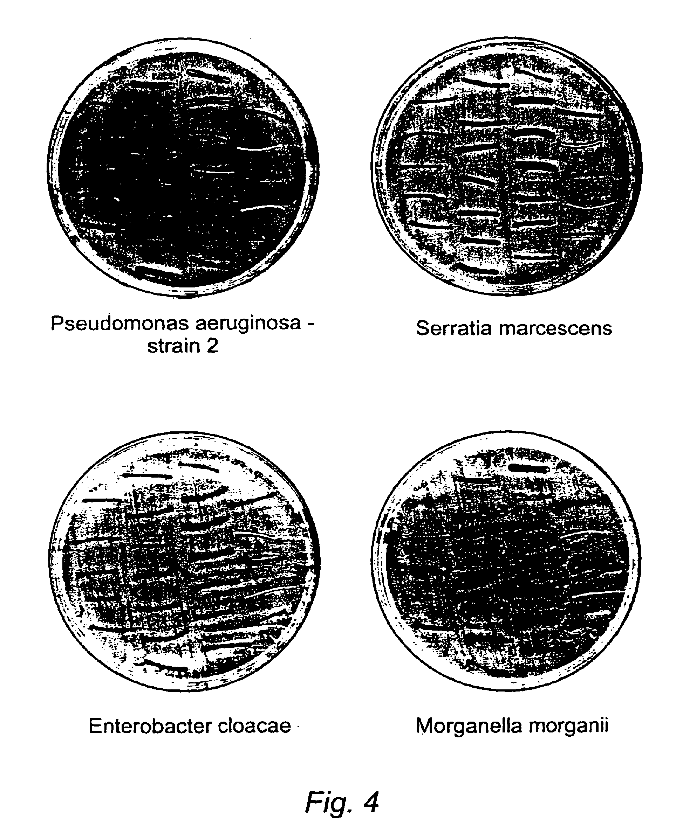

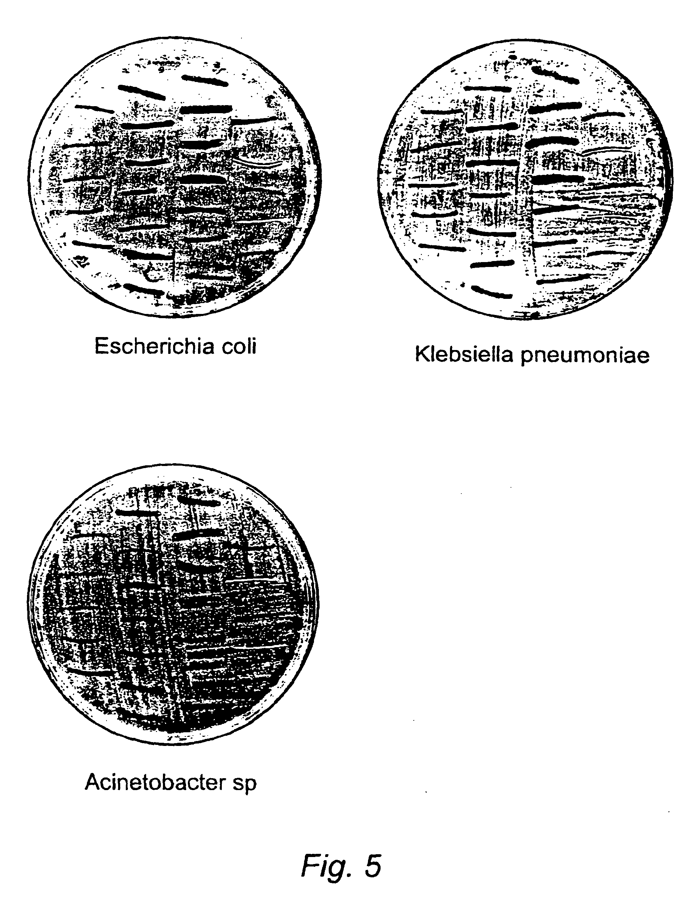

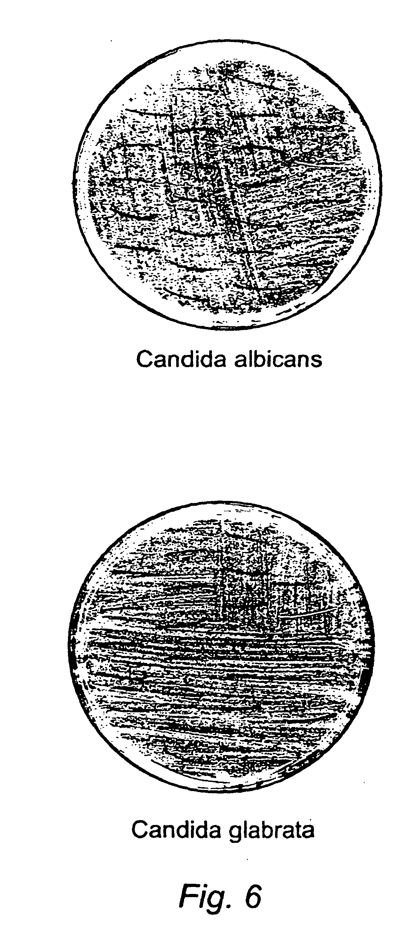

See digitally generated photographic images provided as Figs. 2 to 6 and Table 2.

### Conclusions

### G21-Dacron suture:

Zones of inhibition were seen with all test organisms except *Candida albicans* (organism 16).

### G23-Dacron control suture:

No demonstrable activity.

### G1-G20-violet Polysorb suture:

There was a general trend towards increasing activity with the higher Polysorb suture numbers, with zone sizes plateauing with G14, 15 and 16 followed by a slight decline.

Activity was seen against most organisms in the panel. No zones were seen with two candida isolates (organisms 16 and 17) and the zones for *Stenotrophomonas maltophilia* (organism 7) and *Enterobacter cloacae* (organism 11) tended to be smaller, or absent compared to the other Gram-negative isolates.

Activity against the staphylococcal isolates (organisms 2, 4 and 5) was seen with virtually all sutures. This is of note given the particular importance of staphylococci in the aetiology of stitch abscesses.

The *enterococci* and *streptococci* (organisms 1, 3 and 6) demonstrated the largest zones of inhibition. Interestingly, the control suture (G22) also yielded significant zones for all three organisms, indicating that one of the constituents of the suture has antimicrobial activity in its own right. This constituent must be released from the suture and be able to diffuse through the agar. There is apparent interaction with the components of the test sutures - G2 consistently gave zones smaller than the control.

As will be seen from the digital images (Figs. 2 to 6) the inoculum ranged from semi-confluent to near confluent growth. The Gram-negative organisms tended to a heavier inoculum. Despite the significant challenge, zones of inhibition were seen. At this stage the duration of activity of the test sutures cannot be stated - however, transient contact with the surface of the agar (duration less than 5 seconds) resulted in a small zone of inhibition.

### EXAMPLE 3: Anti-microbial Activity

### Protocol

As for Example 2.

The experiment was performed to confirm the results from the previous experiment, in particular the activity of the G22 control suture against the enterococci and streptococci, and the effect of a lower inoculum on the results from the Gram-negative organisms.

### Results

See Table 3.

**Table 3 : Maximum width of zone of inhibition measured at right angles to the suture (millimetres)**

| **ORGANISM** | | | | |
|---|---|---|---|---|
| | **1 Enterococcus** | **5 Staph 11047** | **6 Gp B strept** | **13 E Coli** |
| **Suture** | | | | |
| **G4** | 9 m | 2 m | 8 m | 0 m |
| **G9** | 9 m | 2.5 m | 9 m | 1 m |
| **G11** | 8 m | 2.5 m | 10 m | 1.5m |
| **G14** | 7.5 m | 3.5 m | 9 m | 2 m |
| **G17** | 8 m | 2.5 m | 8 m | 2 m |
| **G22** | 9 m | 0 m | 12 m | 0 m |

| | | | | |
|---|---|---|---|---|
| **Key**: m - microcolonies present within zone of inhibition | | | | |

### Conclusions

Zone sizes were similar to the results from Example 2. Control suture G22 again demonstrated activity against both enterococci and Gp B streptococci. The zone sizes for the *E coli* using a lighter inoculum were similar to previous results.

### EXAMPLE 4: Controlled Release

### Suture Material

G11 - previously noted to yield a small zone of inhibition with NCTC 11047.

G16 - previously noted to yield a large zone of inhibition with NCTC 11047.

### Test organism

*Staphylococcus epidermidis* NCTC 11047.

### Method

A single plate of Oxoid Iso-sensitest agar (Plate 1) was seeded with the test organism to achieve a semi-confluent growth. Four G11 sutures were applied to one side of the plate, with four G16 sutures on the opposite side. Each suture had been bent to yield a 90° kink in the middle. After 24 hours incubation at 37°C the zones of inhibition at right angles to the sutures were recorded and the sutures were transferred to a freshly seeded Iso-sensitest plate (Plate 2). The kink in the suture ensured that the same aspect of the suture was in contact with the agar surface on each occasion. The new plate was incubated for a further 24 hours and the sutures were removed prior to assessment of zones of inhibition.

### Results

Plate 1 - Each of the G11 sutures yielded a zone of inhibition 1.5 mm in (maximum) width. The G16 sutures yielded zones 2.0 mm in width.

Plate 2 - After transfer to Plate 2, zones of inhibition were not seen for either suture. On removal of the sutures it was observed that there was confluent growth of the test organism under the G11 sutures, but there was inhibition of growth under G16.

### Conclusions

After 24 hours in contact with the agar surface of Plate 1, suture G11 had no demonstrable activity against the test organism on Plate 2. Suture G16 demonstrated marginal activity on Plate 2, with inhibition of growth directly underneath the suture material.

### EXAMPLE 5: Controlled Release

### Suture material

G15 - previously noted to yield a large zone of inhibition with NCTC 11047.

### Test organism

*Staphylococcus epidermidis* NCTC 11047.

### Method

A single plate of Oxoid Iso-sensitest agar was seeded to yield a semi-confluent growth of NCTC 11047. Sixteen sutures were applied with sterile forceps and the plate was incubated at 37°C. At various time intervals sutures were removed. Two sutures were assessed for each time except for "24 hours" where 4 sutures were used. At the end of the 24-hour period the zones of inhibition were assessed and the plate was photographed.

### Results and Conclusions

Suture G15 exhibited activity against the test organism when in contact with the agar surface for only 5 minutes. Activity increases up to the 3 hour point, after which no increased activity is seen.

### EXAMPLE 6: Duration of Anti-microbial effect

### Suture

G16.

### Test organism

*Staphylococcus epidermidis* NCTC 11047.

### Method

An Oxoid Iso-sensitest agar plate was seeded with the test organism to achieve a semi-confluent growth. Six G16 sutures were applied with sterile forceps and the plate was incubated for 24 hours at 37°C. An uninoculated Iso-sensitest plate was also incubated as the Control.

After the initial incubation period each suture was surrounded by a zone of inhibition. Three of the six sutures were then removed. Each zone of inhibition was challenged using a calibrated loop to apply a drop of standardised suspension of test organism. Each drop contained approximately 10⁴ colony forming units. An identical drop was applied to the Control plate. Both plates were then incubated for a further 24 hours and the zones were challenged again, and a further drop was added to the Control plate. The procedure was repeated on a daily basis. The end point of the experiment was when growth appeared in the original zones of inhibition following challenge, or when the Control plate lost the ability to support organism growth due to progressive dehydration. (This was minimised by incubating the plates in an atmosphere with high humidity.)

### Results

Over the thirteen days of the experiment, no growth was seen in any of the zones of inhibition. There was no difference between the zones where the suture remained in place and the zones where the suture had been removed. The experiment was terminated at the 13 day point even though the Control plate continued to support growth of the challenge organism. This was because the test plate appeared to be dehydrating more rapidly, presumably because of the influence of the lawn of growth of NCTC 11047 on its surface.

### Conclusions

Experiment 1 demonstrated that much of the activity of the suture is released in the first 24 hours. Experiment 2 showed that activity is present within 5 minutes of contact with the agar. Experiment 3 illustrates that even though the suture may be depleted, the surrounding area returns antimicrobial activity over a period in excess of one week.

### EXAMPLE 7: Cytotoxity

### 1. Objective

To determine the cytotoxity of a series of suture samples using a standard extraction/elution test, after ISO 10993 part 5.

### 2. Scope

The test procedure applies to all suture samples which were received sterile.

### 3. Equipment and Materials

### 3.1 Equipment

3.1.1 Laminar air flow hood.
3.1.2 Incubator maintained at 37°C/5% carbon dioxide.
3.1.3 Refrigerator at 4°C.
3.1.4 Freezer at -18°C.
3.1.5 Vacuum source.
3.1.6 Phase contrast microscope.

### 3.2 Materials

3.2.1 Sterile plastic-ware pipettes.
3.2.2 Sterile glass pipettes.
3.2.3 24 well sterile dishes.
3.2.4 Surgical grade forceps.
3.2.5 Surgical grade scissors.
3.2.6 Sterile Universal containers.
3.2.7 L929 cell culture line (ATCC NCTC Clone 929).
3.2.8 TCPS negative control.
3.2.9 Natural rubber latex control.
3.2.10 Other control samples were supplied in suture form.

### 4. Procedure

### 4.1 Test sample preparation

4.1.1 Test samples and controls were cut to the appropriate size (see Section 4.2.1) .
4.1.2 Tissue culture polystyrene was employed as a negative control. Natural rubber latex was employed as a positive control. The controls were not in the same physical form as the test material.

### 4.2 Extraction/elution method

All procedures carried out within laminar air flow.
4.2.1 Sutures were prepared to provide a surface area equivalent to 120 cm sq. for each 20 mL of extracting medium.
4.2.2 Suture samples (typically 6 cm in length) were transferred to Sterile Universal containers.
4.2.3 Each container was labelled with the test material code number.
4.2.4 20 mL of mammalian cell culture medium (199) was added to each container.
4.2.5 The containers were placed in the incubator 37°C/5% carbon dioxide for 24 hours.

### 4.3 Cell preparation

4.3.1 A cell subculture was prepared on the same day the extracts were initiated.
4.3.2 Cells were plated into 24 well dishes at a cell concentration of approximately 1 x 10⁵ cells mL. Enough wells were prepared to allow four wells per test sample. 2 mL of serum supplemented medium was added to each well.
4.3.3 The 24-well plates were incubated for 24 hours at 37°C/5% carbon dioxide.

### 4.4 Test procedure

4.4.1 After 24 hours all 24 well plates were examined by phase-contrast microscope (x20 objective lens) to ensure healthy monolayer of >80% confluence.
4.4.2 The culture medium is aspirated.
4.4.3 The Universal containers are removed from the extraction conditions, the pH monitored using phenol red indicator.
4.4.4 2 mL of extracted medium is placed in each well and the plates re-incubated for a 48-hour period.

### 4.5 Interpretation of results

4.5.1 At the conclusion of the incubation period the plates are removed from the incubator and examined under phase contrast microscope using x10 and x20 objective lenses.
4.5.2 Each test and control material was evaluated using the scoring system detailed below.

| **Reactivity Response Table** | | |
|---|---|---|
| **Grade** | **Reactivity** | **Conditions of all cultures** |
| 0 | None | Discrete intracytoplasmic granules; no cell lysis |
| 1 | Slight | No more than 20%, of the cells are round, loosely attached and without intracytoplasmic granules; occasional lysed cells are present |
| 2 | Mild | No more than 50% of the cells are round and devoid of intracytoplasmic granules; extensive cell lysis and empty areas between cells |
| 3 | Moderate | No more than 70% of the cell layers contain rounded cells and/or are lysed |
| 4 | Severe | Nearly complete destruction of the cell layers |

### 4.6 Results

The following table (Table 4) highlights the results obtained following two separate tests: Two readings were taken at each test. In all cases negative control (TCPS) provided a 0 grade and positive control provided a 2 grade.

**Table 4**

| **Material Code** | **Grade Test 1** | | **Test 2** | | **Material Code** | **Grade Test 1** | | **Test 2** | | **Material Code** | **Grade Test 1** | | **Test 2** | |
|---|---|---|---|---|---|---|---|---|---|---|---|---|---|---|
| G1 | 0 | 0 | 0 | 0 | G9 | 0 | 0 | 0 | 0 | G17 | 1 | 0 | 0 | 0 |
| G2 | 0 | 0 | 0 | 0 | G10 | 0 | 0 | 0 | 0 | G18 | 1 | 0 | 0 | 0 |
| G3 | 0 | 0 | 0 | 0 | G11 | 0 | 0 | 0 | 0 | G19 | 1 | 1 | 0 | 0 |
| G4 | 0 | 0 | 0 | 0 | G12 | 0 | 1 | 0 | 0 | G20 | 1 | 1 | 0 | 0 |
| G5 | 0 | 0 | 0 | 0 | G13 | 1 | 1 | 0 | 0 | G21 | 1 | 1 | 0 | 0 |
| G6 | 0 | 1 | 0 | 0 | G14 | 1 | 1 | 0 | 0 | G22 | 2 | 1 | 0 | 1 |
| G7 | 0 | 0 | 0 | 0 | G15 | 1 | 1 | 0 | 0 | G23 | 1 | 1 | 0 | 0 |
| G8 | 0 | 0 | 0 | 0 | G16 | 1 | 1 | 0 | 0 | | | | | |

### Comments

The results as detailed provide a very subjective assessment of material cytotoxity. Where a grade 0 is shown, there was no evidence of toxicity and a confluent healthy monolayer of cells was present. Where there was any evidence of floating cells or morphological abnormality or sub-confluent growth a grade 1 was allocated. It should be noted that floating cells do not necessarily indicate toxicity. It should also be noted that the test 2 indicated less evidence of toxicity than test 1. The extracts (with suture material removed) had been maintained in a frozen state for 72 hours before re-testing.

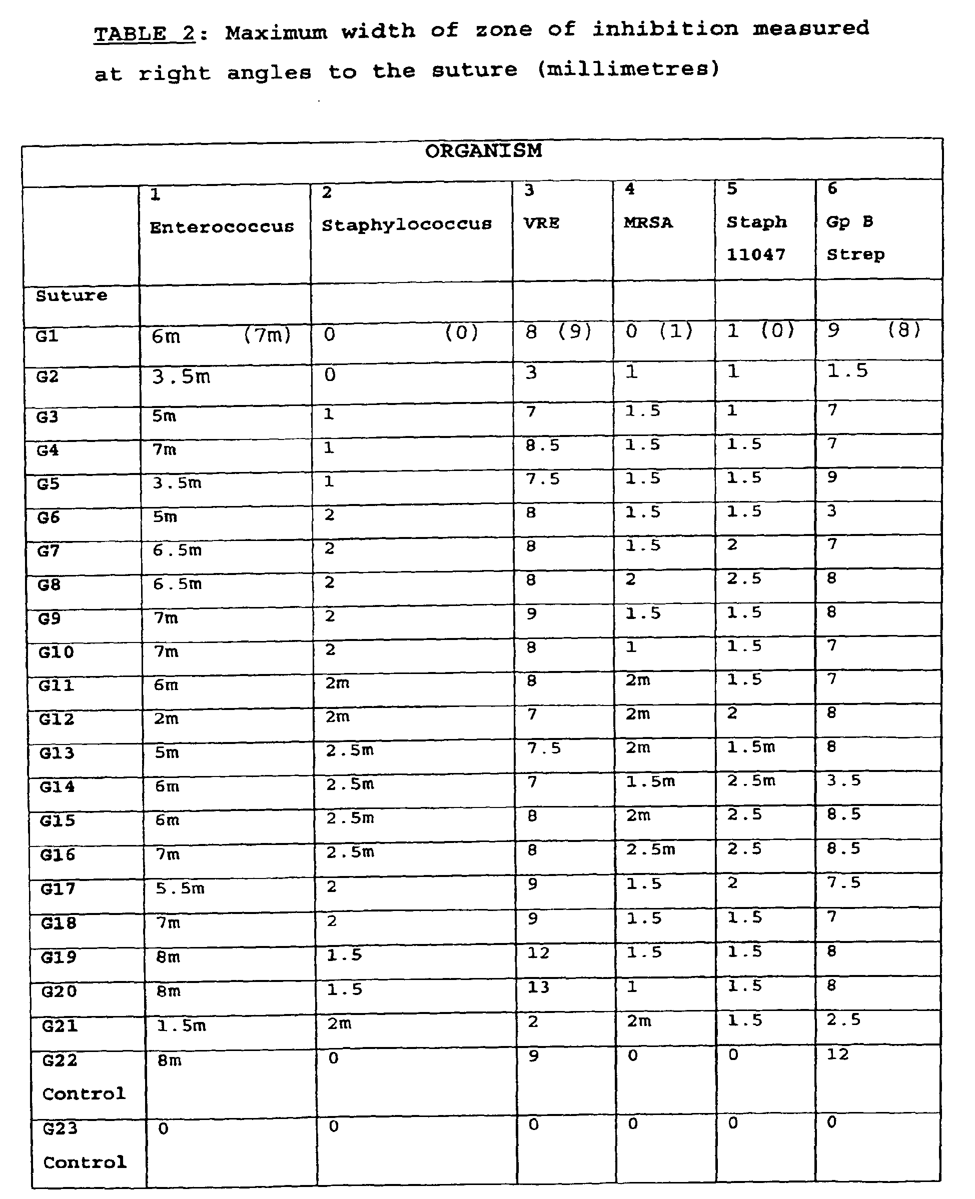

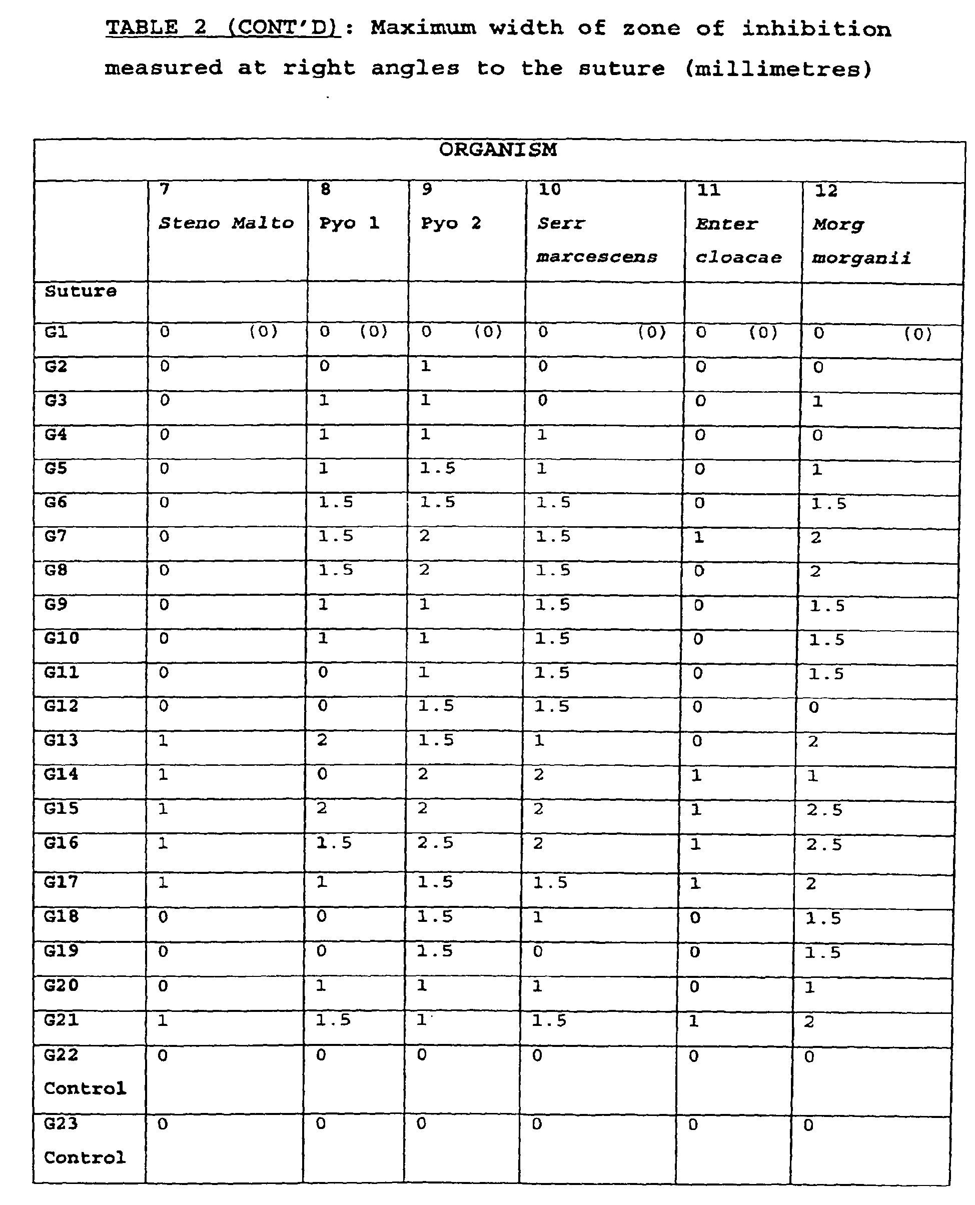

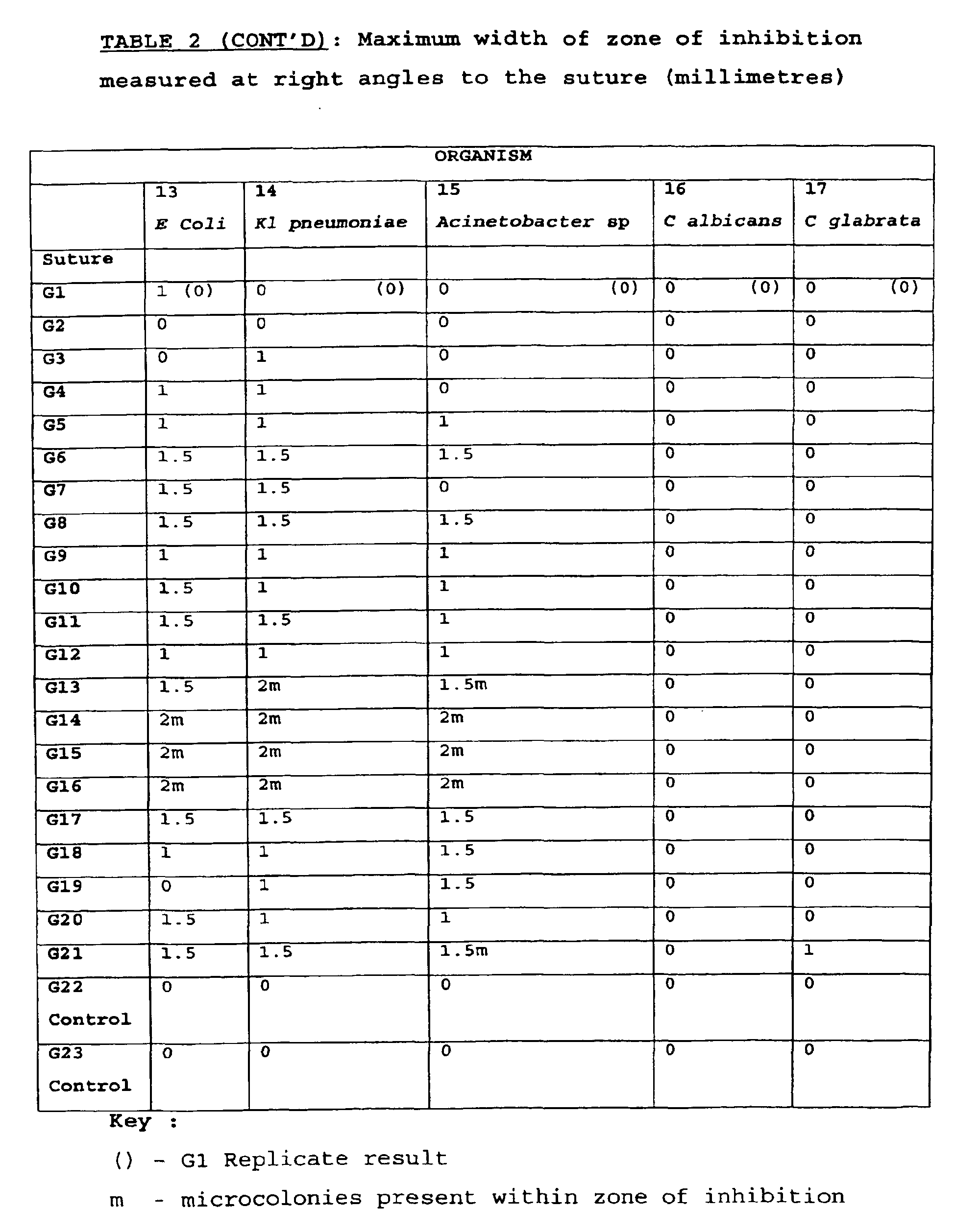

## Claims

1. A surgical suture material having either:
a) an external surface at least partially coated with an anti-microbial composition comprising a water-soluble metal ion-releasing glass as an anti-microbial agent; or
b) a water-soluble metal ion-releasing glass as an anti-microbial agent incorporated therein.

2. The suture material as in Claim 1, wherein said material is selected from silk, polyester, nylon, polypropylene, polyvinylidenefluoride, linen, steel wire, catgut, polyglycolactide, polyamide, fibroin, polyglycolic acid and copolymers thereof.

3. The suture material as claimed in Claim 1 or 2, wherein said material is selected from monofilament, braided multi-filament and twisted multifilament yarns.

4. The suture material as claimed in any one of Claims 1 to 3, wherein said anti-microbial composition is coated on the suture surface.

5. The suture material as claimed in any one of Claims 1 to 4, wherein said anti-microbial agent is biodegradable over a period of time compatible with the timescale of wound healing

6. The suture material as claimed in any one of Claims 1 to 5, wherein said anti-microbial agent is admixed with a coating material.

7. The suture material as claimed in any one of Claims 1 to 6, wherein said glass is in particle form.

8. The suture material as claimed in any one of Claims 1 to 7, wherein said glass enables controlled release of metal ions selected from Ag, Zn, Cu, Mg, Ce, Mn, Bi, Se, Cs and mixtures thereof.

9. The suture material as claimed in Claim 8, wherein said glass enables controlled release of metal ions selected from Ag, Cu, Zn, Mg and mixtures thereof.

10. The suture material as claimed in any one of Claims 1 to 9, wherein said glass releases silver ions.

11. The suture material as claimed in any one of Claims 1 to 10, wherein said glass comprises a source of silver ions which is introduced during manufacture as silver orthophosphate (Ag₃PO₄).

12. The suture material as claimed in any one of Claims 1 to 11, wherein said glass comprises up to 5 mole % of silver.

13. The suture material as claimed in any one of Claims 1 to 12, wherein said glass comprises phosphorous pentoxide (P₂O₅) as a glass former.

14. The suture material as claimed in Claim 13, wherein the mole percentage of phosphorous pentoxide in the glass composition is less than 85%, preferably less than 60% and especially between 30 and 60%.

15. The suture material as claimed in any one of Claims 1 to 14, wherein said glass comprises a glass modifier selected from alkali metals, alkaline earth metals, lanthanoid oxides, lanthanoid carbonates and mixtures thereof.

16. The suture material as claimed in any one of Claims 13 to 15, wherein said glass comprises a glass modifier selected from sodium oxide (Na₂O), potassium oxide (K₂O), magnesium oxide (MgO), zinc oxide (ZnO), calcium oxide (CaO) and mixtures thereof.

17. The suture material as claimed in Claims 15 or 16, wherein the mole percentage of said glass modifier is less than 60%, preferably between 40 and 60%.

18. The suture material as claimed in any one of Claims 1 to 17, wherein said glass comprises a boron containing compound.

19. The suture material as claimed in Claim 18, wherein the mole percentage of said boron containing compound is less than 15%, preferably less than 5%.

20. The suture material as claimed in any one of Claims 1 to 19, wherein said glass comprises an additive compound selected from SiO₂, Al₂O₃, SO₃, sulphate ions (SO₄²⁻), transition metal compounds and mixtures thereof.

21. The suture material as claimed in any one of Claims 1 to 20, wherein said glass has a dissolution rate in water at 38°C in the range from substantially zero to 25mg/cm²/hour.

22. The suture material claimed in Claim 21, wherein said dissolution rate is in the range from 0.01 to 2.0 mg/cm²/hour.

23. The suture material as claimed in any one of Claims 1 to 22 wherein said glass comprises 20-35 mole % Na₂O, 18-30 mole % CaO and 45-60 mole % P₂O₅.

## Patentansprüche

1. Ein chirurgisches Nahtmaterial mit entweder:
a) einer Außenfläche, die zumindest teilweise mit einer antimikrobiellen Zusammensetzung beschichtet ist, welche ein wasserlösliches Metallionen freisetzendes Glas als ein antimikrobielles Agens beinhaltet; oder
b) einem wasserlöslichen Metallionen freisetzenden Glas als antimikrobielles Agens, das darin inkorporiert ist.

2. Nahtmaterial gemäß Anspruch 1, wobei das Material aus Seide, Polyester, Nylon, Polypropylen, Polyvinylidenfluorid, Leinen, Stahldraht, Katgut, Polyglykolaktid, Polyamid, Fibroin, Polyglykolsäure und Copolymeren davon ausgewählt ist.

3. Nahtmaterial gemäß Anspruch 1 oder 2, wobei das Material aus Monofilament-, umsponnenen Multifilament- und verdrillten Multifilamentgarnen ausgewählt ist.

4. Nahtmaterial gemäß einem der Ansprüche 1 bis 3, wobei die antimikrobielle Zusammensetzung auf die Nahtoberfläche aufgetragen wird.

5. Nahtmaterial gemäß einem der Ansprüche 1 bis 4, wobei das antimikrobielle Agens über einen Zeitraum hinweg, der mit der Zeitskala der Wundheilung kompatibel ist, biologisch abbaubar ist.

6. Nahtmaterial gemäß einem der Ansprüche 1 bis 5, wobei das antimikrobielle Agens zu dem Beschichtungsmaterial beigemischt wird.

7. Nahtmaterial gemäß einem der Ansprüche 1 bis 6, wobei das Glas in Partikelform vorliegt.

8. Nahtmaterial gemäß einem der Ansprüche 1 bis 7, wobei das Glas die gesteuerte Freisetzung von Metallionen ermöglicht, welche aus Ag, Zn, Cu, Mg, Ce, Mn, Bi, Se, Cs und Mischungen daraus ausgewählt werden.

9. Nahtmaterial gemäß Anspruch 8, wobei das Glas die gesteuerte Freisetzung von Metallionen ermöglicht, welche aus Ag, Cu, Zn, Mg und Mischungen daraus ausgewählt werden.

10. Nahtmaterial gemäß einem der Ansprüche 1 bis 9, wobei das Glas Silberionen freisetzt.

11. Nahtmaterial gemäß einem der Ansprüche 1 bis 10, wobei das Glas eine Quelle von Silberionen beinhaltet, die während der Herstellung als Silberorthophosphat (Ag₃PO₄) eingeführt wird.

12. Nahtmaterial gemäß einem der Ansprüche 1 bis 11, wobei das Glas bis zu 5 Mol-% Silber beinhaltet.

13. Nahtmaterial gemäß einem der Ansprüche 1 bis 12, wobei das Glas Phosphorpentoxid (P₂O₅) als Glasbildner beinhaltet.

14. Nahtmaterial gemäß Anspruch 13, wobei der Molprozentsatz des Phosphorpentoxids in der Glaszusammensetzung bei weniger als 85 %, vorzugsweise weniger als 60 % und besonders zwischen 30 und 60 % liegt.

15. Nahtmaterial gemäß einem der Ansprüche 1 bis 14, wobei das Glas einen Glasmodifikator beinhaltet, der aus Alkalimetallen, Erdalkalimetallen, Lanthanoidoxiden, Lanthanoidcarbonaten und Mischungen daraus ausgewählt wird.

16. Nahtmaterial gemäß einem der Ansprüche 13 bis 15, wobei das Glas einen Glasmodifikator beinhaltet, der aus Natriumoxid (Na₂O), Kaliumoxid (K₂O), Magnesiumoxid (MgO), Zinkoxid (ZnO), Kalziumoxid (CaO) und Mischungen daraus ausgewählt wird.

17. Nahtmaterial gemäß Anspruch 15 oder 16, wobei der Molprozentsatz des Glasmodifikators bei weniger als 60 %, vorzugsweise zwischen 40 und 60 % liegt.

18. Nahtmaterial gemäß einem der Ansprüche 1 bis 17, wobei das Glas eine Bor enthaltende Verbindung beinhaltet.

19. Nahtmaterial gemäß Anspruch 18, wobei der Molprozentsatz der Bor enthaltenden Verbindung bei weniger als 15 %, vorzugsweise weniger als 5 % liegt.

20. Nahtmaterial gemäß einem der Ansprüche 1 bis 19, wobei das Glas eine Zusatzverbindung beinhaltet, die aus SiO₂, Al₂O₃, SO₃, Sulfationen (SO₄²⁻), Übergangsmetallverbindungen und Mischungen daraus ausgewählt wird.

21. Nahtmaterial gemäß einem der Ansprüche 1 bis 20, wobei das Glas eine Auflösungsgeschwindigkeit in Wasser von 38 °C in dem Bereich von im Wesentlichen null bis 25 mg/cm²/Stunde aufweist.

22. Nahtmaterial gemäß Anspruch 21, wobei die Auflösungsgeschwindigkeit in dem Bereich von 0,01 und 2,0 mg/cm²/Stunde liegt.

23. Nahtmaterial gemäß einem der Ansprüche 1 bis 22, wobei das Glas 20-35 Mol-% Na₂O, 18-30 Mol-% CaO und 45-60 Mol-% P₂O₅ beinhaltet.

## Revendications

1. Un matériau de suture chirurgical ayant soit :
a) une surface externe au moins partiellement enduite d'une composition anti-microbienne comportant un verre hydrosoluble libérant des ions métalliques en tant qu'agent anti-microbien ; soit
b) un verre hydrosoluble libérant des ions métalliques en tant qu'agent anti-microbien incorporé dans celui-ci.

2. Le matériau de suture tel que dans la revendication 1, dans lequel ledit matériau est sélectionné parmi la soie, le polyester, le nylon, le polypropylène, le fluorure de polyvinylidène, le lin, le fil d'acier, le catgut, le polyglycolactide, le polyamide, la fibroïne, l'acide polyglycolique et des copolymères de ceux-ci.

3. Le matériau de suture tel que revendiqué dans la revendication 1 ou la revendication 2, dans lequel ledit matériau est sélectionné parmi des fils monofilaments, multifilaments tressés et multifilaments torsadés.

4. Le matériau de suture tel que revendiqué dans n'importe laquelle des revendications 1 à 3, dans lequel la surface de suture est enduite de ladite composition anti-microbienne.

5. Le matériau de suture tel que revendiqué dans n'importe laquelle des revendications 1 à 4, dans lequel ledit agent anti-microbien est biodégradable sur une période de temps compatible avec l'échelle de temps de cicatrisation de blessure.

6. Le matériau de suture tel que revendiqué dans n'importe laquelle des revendications 1 à 5, dans lequel ledit agent anti-microbien est mélangé avec un matériau d'enduction.

7. Le matériau de suture tel que revendiqué dans n'importe laquelle des revendications 1 à 6, dans lequel ledit verre est sous forme de particules.

8. Le matériau de suture tel que revendiqué dans n'importe laquelle des revendications 1 à 7, dans lequel ledit verre permet la libération contrôlée d'ions métalliques sélectionnés parmi Ag, Zn, Cu, Mg, Ce, Mn, Bi, Se, Cs et des mélanges de ceux-ci.

9. Le matériau de suture tel que revendiqué dans la revendication 8, dans lequel ledit verre permet la libération contrôlée d'ions métalliques sélectionnés parmi Ag, Cu, Zn, Mg et des mélanges de ceux-ci.

10. Le matériau de suture tel que revendiqué dans n'importe laquelle des revendications 1 à 9, dans lequel ledit verre libère des ions argent.

11. Le matériau de suture tel que revendiqué dans n'importe laquelle des revendications 1 à 10, dans lequel ledit verre comporte une source d'ions argent qui est introduite durant la fabrication sous forme d'orthophosphate d'argent (Ag₃PO₄).

12. Le matériau de suture tel que revendiqué dans n'importe laquelle des revendications 1 à 11, dans lequel ledit verre comporte jusqu'à 5 % d'argent en pourcentage molaire.

13. Le matériau de suture tel que revendiqué dans n'importe laquelle des revendications 1 à 12, dans lequel ledit verre comporte du pentoxyde phosphoreux (P₂O₅) en tant que vitrifiant.

14. Le matériau de suture tel que revendiqué dans la revendication 13, dans lequel le pourcentage molaire de pentoxyde phosphoreux dans la composition de verre est inférieur à 85 %, est de préférence inférieur à 60 % et est tout spécialement compris entre 30 et 60 %.

15. Le matériau de suture tel que revendiqué dans n'importe laquelle des revendications 1 à 14, dans lequel ledit verre comporte un modificateur de verre sélectionné parmi des métaux alcalins, des métaux alcalino-terreux, des oxydes de lanthanides, des carbonates de lanthanides et des mélanges de ceux-ci.

16. Le matériau de suture tel que revendiqué dans n'importe laquelle des revendications 13 à 15, dans lequel ledit verre comporte un modificateur de verre sélectionné parmi l'oxyde de sodium (Na₂O), l'oxyde de potassium (K₂O), l'oxyde de magnésium (MgO), l'oxyde de zinc (ZnO), l'oxyde de calcium (CaO) et des mélanges de ceux-ci.

17. Le matériau de suture tel que revendiqué dans les revendications 15 ou 16, dans lequel le pourcentage molaire dudit modificateur de verre est inférieur à 60 %, de préférence entre 40 et 60 %.

18. Le matériau de suture tel que revendiqué dans n'importe laquelle des revendications 1 à 17, dans lequel ledit verre comporte un composé contenant du bore.

19. Le matériau de suture tel que revendiqué dans la revendication 18, dans lequel le pourcentage molaire dudit composé contenant du bore est inférieur à 15 %, de préférence inférieur à 5 %.

20. Le matériau de suture tel que revendiqué dans n'importe laquelle des revendications 1 à 19, dans lequel ledit verre comporte un composé formant additif sélectionné parmi SiO₂, Al₂O₃, SO₃, des ions sulfate (SO₄²⁻), des composés formant métaux de transition et des mélanges de ceux-ci.

21. Le matériau de suture tel que revendiqué dans n'importe laquelle des revendications 1 à 20, dans lequel ledit verre a une vitesse de dissolution dans l'eau à 38 °C dans la gamme allant de substantiellement zéro à 25 mg/cm²/heure.

22. Le matériau de suture revendiqué dans la revendication 21, dans lequel ladite vitesse de dissolution se trouve dans la gamme allant de 0,01 à 2,0 mg/cm²/heure.

23. Le matériau de suture tel que revendiqué dans n'importe laquelle des revendications 1 à 22, dans lequel ledit verre comporte de 20 à 35 % de Na₂O en pourcentage molaire, de 18 à 30 % de CaO en pourcentage molaire et de 45 à 60 % de P₂O₅ en pourcentage molaire.
